Europäisches Patentamt

European Patent Office

Office européen des brevets

(19)

(11) Numéro de publication : **0 381 736 B1**

# (12) FASCICULE DE BREVET EUROPEEN

(45) Date de publication du fascicule du brevet :
23.12.92 Bulletin 92/52

(51) Int. Cl.⁵ : **A61M 5/14**, G01N 35/00

(21) Numéro de dépôt : **89908712.6**

(22) Date de dépôt : **20.07.89**

(86) Numéro de dépôt international :
**PCT/FR89/00380**

(87) Numéro de publication internationale :
**WO 90/00909 08.02.90 Gazette 90/04**

(54) **PANCREAS ARTIFICIEL.**

(30) Priorité : **21.07.88 FR 8810036**

(43) Date de publication de la demande :
**16.08.90 Bulletin 90/33**

(45) Mention de la délivrance du brevet :
**23.12.92 Bulletin 92/52**

(84) Etats contractants désignés :
**AT BE CH DE FR GB IT LI LU NL SE**

(56) Documents cités :
EP-A- 0 064 691
DD-A- 222 124
DE-A- 2 849 367
US-A- 3 994 593
US-A- 4 717 546
Life Support Systems, volume 1, no.1, jan-
vier-mars 1983, European Society for Artificial
Organs, (Eastbourne, East Sussex,GB), J.
Mirouze et al.: "Clinical experience in human
diabetics with portable and implantable insulin minipumps", pages 39-49

(73) Titulaire : **SOCIETE D'APPLICATIONS DES
TECHNIQUES PHOTONIQUES
La Clais
F-35740 Pace (FR)**

(72) Inventeur : **GUEGAN, Alain
3, rue de Merville
F-56100 Lorient (FR)**
Inventeur : **GY, Jules, Jean-Pierre
La Clais
F-35740 Pace (FR)**
Inventeur : **HESPEL, Jean-Pierre
3, impasse René-Bazin
F-35700 Rennes (FR)**
Inventeur : **ROUYER, Philippe
7, avenue Pinault
F-35740 Pace (FR)**

(74) Mandataire : **Martin, Jean-Jacques et al
Cabinet REGIMBEAU 11, rue Franz Heller
Centre d'Affaires Patton B.P. 19107
F-35019 Rennes Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un pancréas artificiel.

On sait que le pancréas a une double fonction. La première est une fonction digestive, par les acini qu'il renferme, le pancréas secrétant des ferments digestifs dans le duodénum. La seconde est une fonction régulatrice, le pancréas stabilisant le taux de glucose présent dans le sang grâce aux îlots de Langherhans, qui secrètent au moins quatre hormones.

Le pancréas a donc pour rôle de stabiliser la glycémie sanguine (taux de glucose sanguin). C'est à cette fonction que se rapporte la présente invention. Le pancréas réalise cela à partir de plusieurs hormones dont la principale est l'insuline. Quel que soit le glucose absorbé, la glycémie sanguine ne doit pas varier énormément autour d'une valeur moyenne, laquelle est de l'ordre de 0,9 g/l (grammes par litre) ; c'est le cas des sujets non diabétiques.

Dans le cas d'un sujet bien portant, la glycémie oscille entre 0,5 et 1,5 g/l suivant le sujet et le moment de la journée.

Un sujet diabétique est une personne dont le taux de glucose sanguin n'est pas stabilisé par l'organisme. Lorsqu'il y a diabète, la glycémie peut dépasser 1,5 g/l (hyperglycémie) ou être inférieure à 0,5 g/l (hypoglycémie).

Le pancréas artificiel qui fait l'objet de l'invention est un dispositif extracorporel (non destiné à être implanté) qui se propose de ramener le taux de glucose (glycémie) à une va-leur normale, par injection programmée d'insuline et/ou de glucose.

Le pancréas artificiel se présente comme étant un système en boucle fermée où le taux de glucose est la grandeur à asservir. Le système agit en injectant l'insuline ou le glucose dont le débit a été calculé à partir de la glycémie d'un échantillon sanguin, ceci au moyen d'un algorithme préalablement défini par des thérapeutes et intégré dans le programme.

Ce pancréas artificiel est destiné non pas à être utilisé en permanence chez un individu diabétique, mais à être utilisé temporairement, par exemple durant une période de l'ordre d'une journée, pour examiner un patient afin de connaître ses besoins et ses réactions à l'injection d'insuline ou de glucose, ceci de manière à pouvoir déterminer avec précision le traitement qui lui convient et qui lui sera prescrit ensuite.

Dans les dispositifs connus de ce genre, la mesure de la glycémie se fait généralement par la méthode traditionnelle (chimie humide), ce qui est long, fastidieux, et requiert du personnel qualifié ; des méthodes plus modernes ont été proposées, notamment par le document EP-A-098 592 - lequel propose un pancréas artificiel dans lequel la mesure de la glycémie est effectuée par voie électro-polarographique, ce qui permet d'opérer de manière automatique ; toutefois ce matériel apparaît relativement sophistiqué, très coûteux et délicat à utiliser.

La présente invention vise à résoudre ces problèmes en proposant un dispositif du type évoqué dont l'unité de mesure de la glycémie soit robuste et durable, relativement bon marché, donne des résultats précis et fiables, se prête à un automatisme total de l'opération. Ce résultat est essentiellement obtenu grâce au fait que la technique de mesure mise en oeuvre est une technique éprouvée de chimie sèche, qui consiste à lire par voie optique la couleur prise par la zone réactive à la glycémie d'une bandelette, zone sur laquelle l'échantillon sanguin a préalablement été déposé sous forme d'une goutte. Ce type de mesure est couramment pratiqué périodiquement par les diabétiques qui peuvent ainsi contrôler eux-mêmes leur niveau de glycémie.

C'est pourquoi l'invention a pour principal objectif d'intégrer cette technique de mesure éprouvée dans un pancréas artificiel, ceci en en automatisant le processus.

Le pancréas artificiel objet de l'invention comprend une unité de prélèvement automatique et périodique d'échantillons de sang dans le corps d'un patient, une unité de mesure du taux de glucose présent dant cet échantillon (glycémie), une unité d'injection d'une dose d'insuline ou de glucose dans le corps du patient, et une unité de calcul et de traitement informatique apte à déterminer la quantité d'insuline ou de glucose devant être administrée au patient en fonction du taux de glucose mesuré par l'unité de mesure, et à commander de manière correspondante l'unité d'injection. Ce pancréas artificiel est caractérisé en ce que la mesure de la glycémie est effectuée par lecture de la couleur de la zone d'une bandelette qui est réactive à la glycémie, l'unité de mesure comprenant à cet effet :

a) Un poste de distribution de bandelettes ;

b) un poste dans lequel une goutte de sang fournie par ladite unité de prélèvement est déposée sur la zone réactive d'une bandelette ;

c) un poste d'essuyage de la zone ;

d) un poste de lecture optique de la couleur prise par cette zone ;

e) un poste d'évacuation de la bandelette ;

f) un dispostif de transport apte à acheminer individuellement les bandelettes de poste en poste.

Par ailleurs, selon un certain nombre de caractéristiques avantageuses (mais non limitatives) :

- le dispositif de transport comprend une pince dont les mâchoires sont adaptées pour saisir et retenir une bandelette, ladite pince étant montée mobile en translation selon un axe sensiblement horizontal dans une tourelle rotative d'axe vertical ;

- le poste de distribution des bandelettes comprend un réceptacle dans lequel les bandelettes sont empilées horizontalement sous une certaine pression, une molette d'entraînement rotative prévue au niveau du fond de ce réceptacle étant adaptée pour faire sortir du réceptacle, par une ouverture appropriée, la bandelette inférieure de la pile ;

- l'une des mâchoires de ladite pince est munie d'une languette élastique apte à bloquer la bandelette pour l'empêcher de ressortir de la pince ;
- le poste dans lequel une goutte de sang est déposée sur la zone réactive de la bandelette comprend une plaque-support traversée par un petit conduit d'amenée du sang et un élément d'appui de la bandelette disposé à l'aplomb dudit petit conduit d'amenée ;
- le poste d'essuyage comprend un bâti équipé d'une paire de bobines respectivement distributrice et réceptrice d'une bande de papier absorbant, des moyens étant prévus pour entraîner en rotation l'une au moins des bobines sur une certaine course, et ainsi faire défiler la bande ;
- la bande est guidée par des galets pour passer entre un sabot presseur inférieur et un sabot presseur supérieur, la zone réactive de la bandelette venant se positionner entre ces deux sabots, avec la bande appliquée contre sa zone réactive ;
- la bande de papier absorbant et le sabot presseur supérieur sont portés par une plaque qui est articulée sur le bâti autour d'un axe horizontal, cette plaque étant solidaire d'une came apte à coopérer avec un organe de commande équipant la pince pour provoquer le soulèvement temporaire du sabot supérieur et de la bande lors de la mise en place de la zone réactive de la bandelette entre les deux sabots, et lors du retrait de cette bandelette ;
- le poste de lecture optique de la couleur prise par la zone réactive de la bandelette comprend un boîtier de lecture pourvu d'une fente tournée vers le dispositif transporteur et orientée de manière à se trouver sur la trajectoire de la bandelette ;
- la pince comprend une mâchoire supérieure fixe et une mâchoire inférieure articulée autour d'un axe horizontal, la fermeture de la pince étant normalement assurée par l'appui de cette mâchoire inférieure sur la platine de montage de l'ensemble des postes ;
- le poste d'évacuation de la bandelette comprend une plaquette inclinée qui est munie d'une languette de retenue de la bandelette, cette plaquette étant disposée à proximité d'une ouverture ménagée dans la platine de montage, cette ouverture réalisant d'une part l'ouverture de la pince par basculement de la mâchoire inférieure et permettant d'autre part le passage des bandelettes évacuées, avantageusement vers un récipient récepteur.

D'autres caractéristiques et avantages de l'invention apparaîtront de la description et des dessins annexés qui en présentent un mode de réalisation préférentiel, adapté à l'injection d'insuline.

Sur ces dessins :
- la figure 1 est une vue générale schématique de l'ensemble du système de pancréas artificiel qui fait l'objet de l'invention ;
- la figure 2 représente vue de côté et en coupe longitudinale, le dispositif de transport ;
- la figure 3 est une coupe transversale du dispositif de la figure 2 ;
- la figure 4 représente, en pespective, une bandelette réactive ;
- la figure 5 représente le poste de distribution des bandelettes ;
- la figure 5a est une vue analogue à la figure 5, montrant de quelle manière s'opère la distribution d'une bandelette ;
- la figure 6 est une vue de côté schématique du poste dans lequel une goutte de sang est déposée sur la zone réactive d'une bandelette ;
- la figure 7 est une vue de côté schématique du poste d'essuyage ;
- la figure 8 est une vue analogue à la figure 7, qui montre de quelle manière se fait le soulèvement de la tête d'essuyage lors de la mise en place d'une bandelette à ce poste ;
- la figure 9 est une vue de dessus schématique du poste de lecture optique de la couleur prise par la zone réactive d'une bandelette ;
- la figure 10 est une vue schématique du poste d'évacuation des bandelettes usagées, les figures 11 et 12 étant des vues similaires montrant plusieurs étapes du processus d'évacuation ;
- la figure 13 est une vue schématique du cathéter double permettant le prélèvement d'un échantillon de sang hépariné ;
- la figure 14 est une vue de dessus schématique du dispositif pousse-seringue.

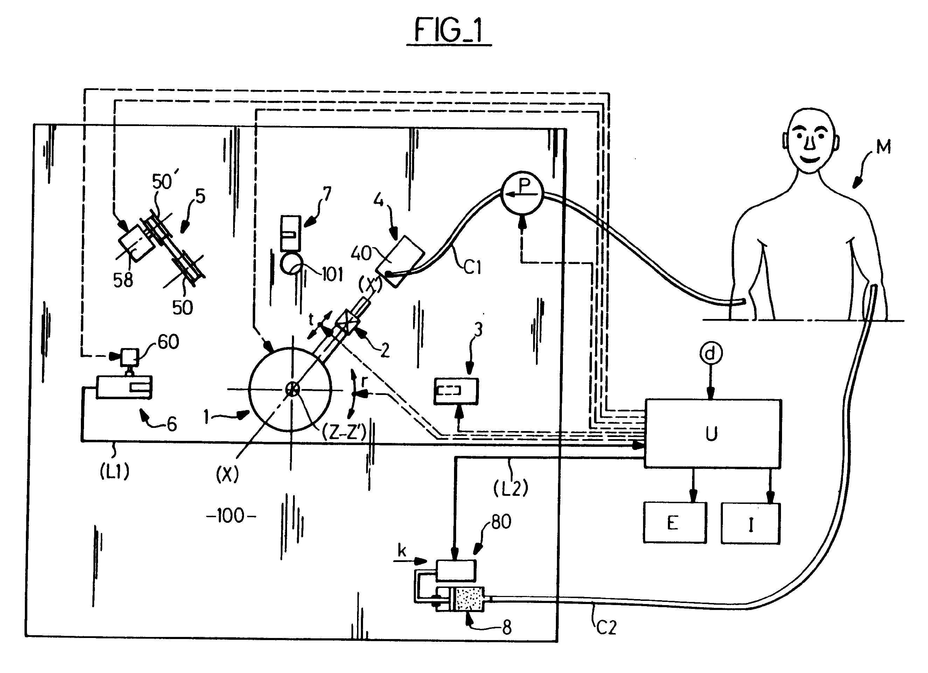

La figure 1 est une vue générale du pancréas artificiel selon l'invention. Ce dispositif comprend un socle ou platine de montage 100 sur lequel sont montés les différents postes constitutifs de l'unité de mesure de la glycémie, le dispositif de transport des bandelettes de poste en poste, ainsi que l'unité d'injection de l'insuline le patient (sujet diabétique) est désigné par la référence (M). Une unité de prélèvement automatique et périodique d'échantillons de sang dans le corps de ce patient comprend un cathéter $(C_1)$ qui est enfoncé dans l'un des bras du patient pour en prélever le sang, le pompage de celui-ci étant réalisé par une pompe (P) par exemple de type péristaltique.

L'unité d'injection d'une dose d'insuline comprend une seringue 8 contenant une certaine quantité d'insuline, qui est reliée par un second cathéter $(C_2)$ à l'autre bras du patient (M). La seringue est actionnée par un pousse-seringue 80, de type connu, apte à délivrer une dose prédéterminée d'insuline, par déplacement du piston de la seringue sur une certaine course bien définie (flèche k, figure 1).

Sur la platine 100, qui est une plaque métallique stable, sont fixés par des moyens appropriés tel que des vis par exemple, les différents postes constitutifs de l'unité de mesure. Ces postes sont :
- un poste 3 de distribution de bandelettes, un poste 4 auquel débouche le premier cathéter (C₁), dont le rôle est de déposer une goutte de sang sur la zone réactive d'une bandelette ;
- un poste 5 d'essuyage de la zone réactive de la bandelette ;
- un poste 6 de lecture optique de la couleur prise par la zone réactive ;
- un poste 7 d'évacuation de la bandelette.

On notera qu'au poste 4 est prévue une plaque horizontale 40 à laquelle est fixée l'extrémité aval du cathéter (C₁) ; on remarquera que devant le poste d'évacuation 7, est prévue une ouverture 101 traversant la platine 100 ; on remarquera aussi que le poste d'essuyage 5 comprend deux bobines 50, 50′ dont l'une (50′) est entraînée en rotation par des moyens appropriés 58 tel qu'un moto-réducteur électrique ; on notera enfin que le poste de lecture 6 est équipé d'un organe d'actionnement, tel qu'un électro-aimant 60, adapté pour agir sur un interrupteur du dispositif de lecture.

Dans la partie centrale de la platine 100 est monté un dispositif de transport qui comprend une partie rotative 1 apte à tourner autour d'un axe vertical (Z-Z′), cette partie portant une pince 2 qui est mobile en translation suivant un axe horizontal (parallèle à la platine 100), radialement par rapport à l'axe (Z-Z′). Cet axe, le long duquel se déplace la pince 2, est référencé (X-X′).

La possibilité de rotation du dispositif de transport, qui correspond au pivotement de la partie 1, dans un sens ou dans l'autre, autour de l'axe (Z-Z′) est figurée par la double flèche (r), tandis que la possibilité de translation de la pince 2 suivant l'axe (X-X′) est figurée par la double flèche (t).

Les différents postes 3, 4, 7, 5 et 6 sont disposés sensiblement sur un cercle fictif d'axe (Z-Z′). Chacun de ces postes est donc accessible à la pince 2, qui peut y transporter une bandelette, après rotation de la partie 1 sur un angle approprié et translation de la pince 2 en direction du poste concerné.

On a désigné par la référence (U) une unité de calcul et de traitement informatique qui est apte à déterminer la quantité d'insuline devant être administré au patient en fonction du taux de glucose mesuré par l'unité de mesure. Il est possible d'introduire, au moyen d'un clavier approprié, dans cette unité, des données (d) relatives au patient (M). La détermination de la quantité d'insuline voulue se fait suivant un algorithme approprié, préalablement mis au point par le thérapeute. Cette unité de calcul et de traitement est avantageusement reliée à un écran de visualisation (E) et à une imprimante (I). Elle reçoit comme principale information la lecture de la glycémie faite au poste 6, les signaux électriques correspondants étant transmis par une ligne (L₁). Après traitement de cette information, l'unité (U) envoie à l'unité d'injection d'insuline, un ordre adéquat, correspondant à la dose qui doit être administrée au patient (M) par le cathéter (C₂) ; cet ordre transite par un branchement référencé (L₂).

Parallèlement, par des moyens à la portée de l'homme du métier, qui ne seront pas développés ici, l'unité de calcul et de traitement informatique, va piloter, selon un séquencement et des temporisations bien définis, la mise en route du transporteur 1,2, et celle des différents postes 3, 4, 5, 6, 7 de l'unité de mesure.

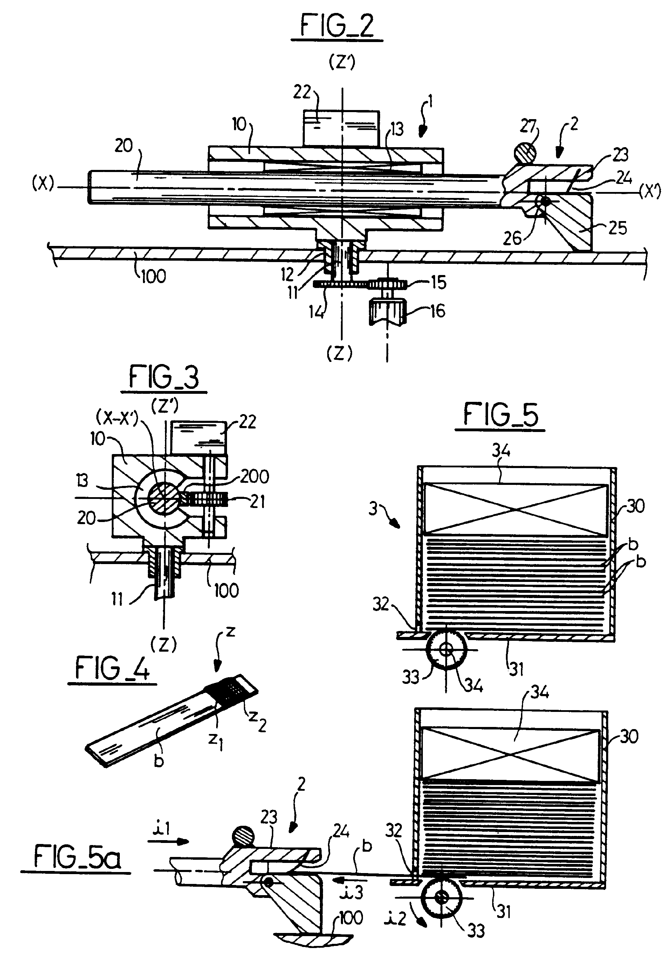

Les figures 2 et 3 représentent le transporteur. Comme on l'a déjà dit, celui-ci comprend une partie 1 apte à tourner autour d'un axe horizontal (Z-Z′), et une partie 2 formant pince, apte à se déplacer en translation le long d'un axe (X-X′) (axe qui est lui-même mobile autour de l'axe (Z-Z′) précité).

La partie 1 comprend un corps principal ou tourelle 10 qui est porté par un arbre 11 d'axe (Z-Z′) guidé en rotation dans un manchon 12 qui traverse la platine 100. Sous la platine, l'arbre 11 porte un pignon 14 qui est en prise avec un autre pignon 15 monté à la sortie d'un petit moto-réducteur électrique 16. La mise en rotation dans un sens ou dans l'autre du moto-réducteur 16 entraîne la rotation correspondante de la tourelle 10 autour de l'axe (Z-Z′). Le moto-réducteur 16 est monté sous la platine 100 par des moyens appropriés qui n'ont pas été représentés dans un seul but de simplification.

La partie 2 formant pince comprend une tige 20, par exemple cylindrique, qui est guidée en translation dans la tourelle 10, par l'intermédiaire de moyens de guidage appropriés 13, par exemple à billes ; l'une des extrémités de la tige 20 porte une tête de préhension - ou pince - constituée par une mâchoire supérieure fixe 23 et une mâchoire inférieure 25 qui est articulée sur la tige 20 autour d'un axe horizontal 26. La partie inférieure de la mâchoire mobile 25 vient en appui contre le dessus de la platine 100, de manière à maintenir la pince fermée. La partie fixe 23 est munie d'une languette élastique 24, par exemple en acier ressort, qui est disposé en oblique comme on le voit à la figure 2, et vient traverser l'intervalle séparant les parties fixe 23 et mobile 25.

Sur le dessus de la tête de préhension est fixé en outre un organe de commande de came 27, dont le rôle sera expliqué plus loin ; l'organe 27 est par exemple une tige cylindrique soudée.

Le long de la tige 20, par exemple sur l'un des côtés de celle-ci est fixée une denture 200 qui constitue une crémaillère ; celle-ci engrène avec un pignon 21 solidaire d'un arbre vertical qui est guidé convenablement dans la tourelle 10 (voir figure 3). Le pignon 21 est entraîné en rotation, dans un sens ou dans l'autre, par un petit moto-réducteur électrique 22 monté sur le dessus de la tourelle 10. On comprend qu'en faisant tourner ce moto-réducteur dans un sens ou dans l'autre, on réalise le déplacement de la tige 20 selon l'axe (X-X') dans un sens ou dans l'autre, c'est-à-dire en faisant reculer la tête de préhension en direction de la tourelle (rétraction), ou au contraire en la faisant avancer vers l'extérieur (extension).

La figure 4 représente le type de bandelette pouvant être utilisé dans l'unité de mesure qui fait l'objet de l'invention. La bandelette (b) est un petit rectangle allongé, en carton ou en matière plastique de faible épaisseur ; à l'extrémité de la bandelette, sur l'une de ses deux faces, se trouve une zone (z) comportant une substance réactive à la glycémie ; la particularité de cette substance est de changer de couleur de manière continue en fonction du taux de glucose du liquide - et notamment du sang - dont elle est imprégnée. Le changement de couleur permet de déterminer avec une grande précision ce taux de glucose. En fait il est prévu deux zones distinctes réactives ($z_1$, $z_2$), ce qui permet d'améliorer encore la précision et la fiabilité de la lecture. Des bandelettes de ce type pour test glycémique sont commercialisées notamment par la société BMP (Boehringer Mannheim Pharma) sous le nom commercial "Haemo-Glukotest".

La figure 5 représente le poste 3 de distribution de bandelettes (b). A ce poste est prévu un réceptacle 30, qui a la forme d'une boîte dans laquelle sont stockées, en position empilées les unes sur les autres, des bandelettes prêtes à l'emploi (b).

Une masselotte 34 placée sur l'empilement de bandelettes réalise un certain pressage de celles-ci contre le fond 31 du réceptacle. Ce fond est équipé d'un rouleau distributeur 33, qui a de préférence la forme d'une molette ; celle-ci est portée par un arbre horizontal 34 qui est entraîné par un petit moto-réducteur électrique non représenté. Le haut de la molette 33 vient à fleur du fond 31, et porte contre le dessous de la bandelette inférieure se trouvant dans le réceptacle 30. Les zones réactives qui n'ont pas été représentées sur les figures 5 et 5a sont situées aux extrémités situées vers la droite de ces figures, et sont tournées vers le haut.

Du côté gauche (où est située la molette) est prévue une petite ouverture 32 qui est disposée en vis à vis de la bandelette inférieure.

La figure 5a montre comment se fait la distribution d'une bandelette du réceptacle 30 à la pince 2.

La pince 2 ayant été positionnée, par rotation appropriée de la tourelle 10, en regard du réceptacle 30, la pince 2 est déplacée (par mise en route du moto réducteur 22) vers l'extérieur, pour venir à une faible distance de celui-ci. La hauteur du haut de la mâchoire 25 par rapport au-dessus de la platine 100 correspond à la hauteur de l'ouverture 32 par rapport à cette même platine. Ce rapprochement de la pince 2 est figuré par la flèche ($i_1$) à la figure 5a. La pince 2 se trouve

vant dans une position convenable, le moto-réducteur commandant la rotation de la molette 33 est mise en route, et la molette tourne dans le sens de la flèche ($i_2$). En raison de la pression exercée par la masselotte 34, la dernière bandelette (b) de la pile est entraînée par friction par la molette, et ressort à travers l'ouverture 32 hors du réceptacle 30 ; son extrémité libre passe alors sous la lame élastique 24 (qui fléchit) et la bandelette vient se coincer automatiquement entre les deux mâchoires (flèche $i_3$). La bandelette est alors parfaitement solidaire de la pince 2, sa zone réactive (z) étant dirigée vers l'extérieur et tournée vers le haut.

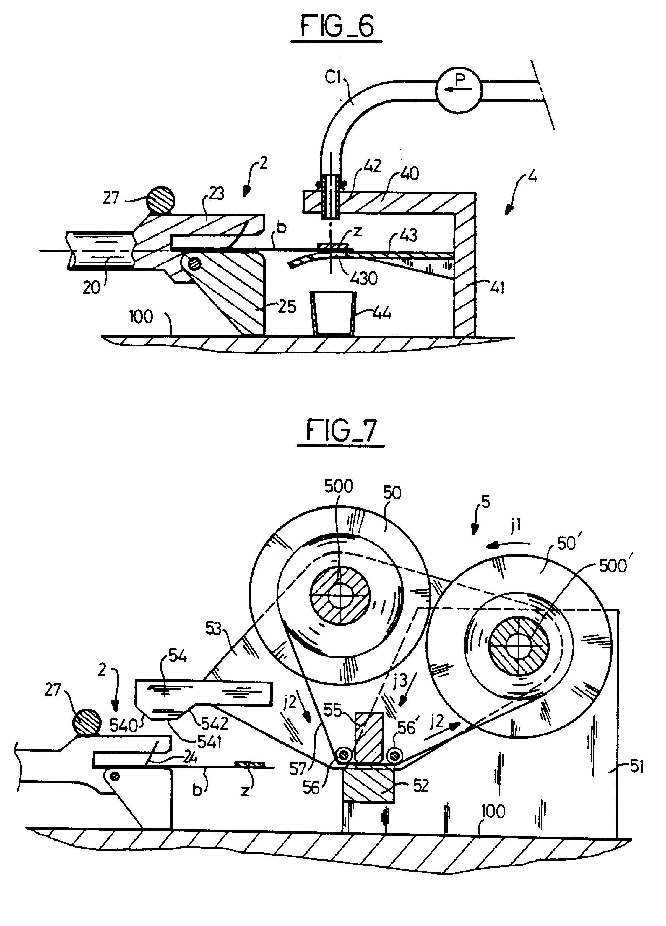

La figure 6 représente le poste auquel une goutte de sang va être déposée sur la zone réactive de la bandelette portée par la pince 2. Ce poste comprend une plaque support 40 sensiblement horizontal, qui est fixé à la platine 100 par des moyens appropriés, par l'intermédiaire d'une potence 41. La plaque 40 est traversée par un petit conduit 42 sur lequel est branchée l'extrémité du cathéter ($C_1$). Sous la plaque 40 est disposée un élément d'appui 43 ayant par exemple la forme d'une petite plaquette, également solidaire de la potence 41. A l'aplomb du conduit 42 il est prévu dans la plaquette 43 une ouverture 430 sous laquelle est disposé un petit récipient 44. Le transporteur est agencé de telle manière que la pince 2 peut amener une bandelette (b) au poste 4 en positionnant la zone réactive (Z) juste au-dessous du conduit 42. La présence de l'ouverture 430 évite de salir l'élément d'appui 43 en cas d'amenée de sang en l'absence de bandelette (b) au poste 4.

Le poste d'essuyage 5 représenté à la figure 7 comprend un bâti fixe 51 (porté par la platine 100) qui supporte une plaque articulée 53 ; sur cette dernière sont montées deux bobines respectivement distributrice 50 et réceptrice 50' d'axe horizontal 500, respectivement 500' ; ce même axe 500' sert avantageusement à l'articulation de la pièce 53 sur le bâti 51.

Le dispositif d'essuyage comporte deux sabots presseurs l'un inférieur fixe 52 (porté par le bâti 51) l'autre supérieur mobile 55 (porté par la plaque articulée 53). De part et d'autre du sabot supérieur 55 sont prévus des galets de renvoi 56, 56' pour une bande de papier absorbant (papier à base de cellulose). Cette bande - ou ruban - est déroulée de la bobine 50 et enroulée sur la bobine 50', après passage sur les galets de renvoi 56, 56', entre les deux sabots 55, 52. La bobine réceptrice 50' est entraînée en rotation par un moto-réducteur approprié 58, qui n'a pas été représenté à la figure 7 pour ne pas l'alourdir inutilement. La mise en route de ce moto-réducteur permet de faire tourner la bobine 50' dans le sens de la flèche ($j_1$), et corrélativement de faire défiler la bande de papier absorbant dans le sens des flèches ($j_2$).

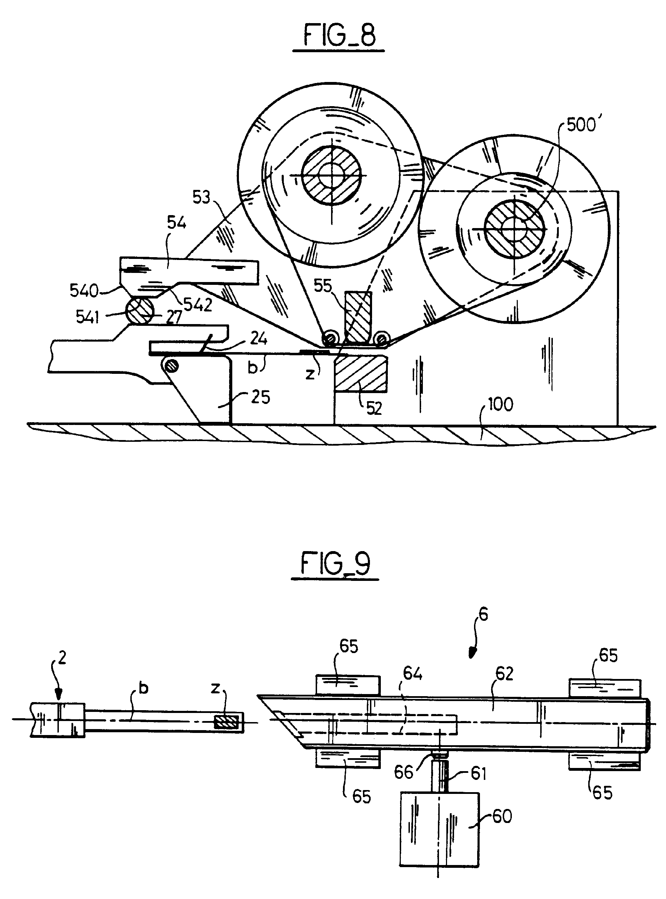

Du côté tourné vers le centre de la platine 100, c'est-à-dire vers le transporteur 1,2, la plaque 53 porte une came profilée 54. Celle-ci présente un bord avant biseauté 540, une partie inférieure sensiblement horizontale 541, et un bord arrière biseauté 542. Ces surfaces sont conçues pour coopérer avec l'organe 27 prévu sur le dessus de la pince 2 afin de provoquer, lors de l'approche de la pince chargée d'une bandelette, le soulèvement provisoire de la plaque 53 de manière à autoriser la mise en place de l'extrémité de la bandelette sous la bande 57, entre les sabots presseurs 55, 52. L'observation de la figure 8 permet aisément de comprendre cette opération. Une fois que ces surfaces de came ont été franchies, la plaque 53 bascule vers le bas par son propre poids, et le sabot presseur 55 vient appliquer la bande 57 contre la zone (z), la bandelette étant elle-même appliquée contre le sabot inférieur 52. De même, le retrait de la bandelette est possible par suite du recul de la pince 2, le bord de came arrière 542 réalisant alors le soulèvement de l'ensemble.

La figure 9 représente en vue de dessus le poste 6 de lecture. Ce poste est équipé d'un boîtier 62 pourvu d'une fente 64 qui est tournée vers le dispositif transporteur et orientée de manière à se trouver sur la trajectoire de la bandelette (b) lorsque celle-ci est amenée à ce poste. Le dispositif de lecture 62 est un dispositif en soi connu, par exemple du type commercialisé sous le nom "REFLOLUX F" (marque déposée) par la société BMP (Boehringer Mannheim Pharma).

Le boîtier 62 est correctement maintenu en place sur la platine 100 par des moyens appropriés tels que des mors de serrage 65. Il est prévu sur ce boîtier un interrupteur 66 pour la mise en route du lecteur.

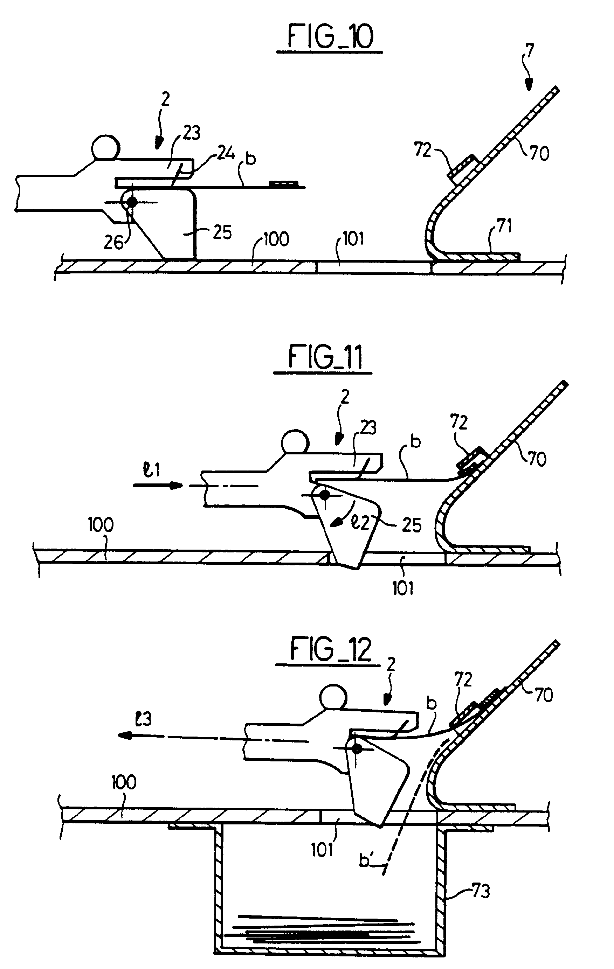

Les figures 10 à 12 représentent le poste d'évacuation de la bandelette usagée, après lecture. Ce poste 7 est équipé d'une plaquette inclinée 70 pourvue d'un socle 71 qui est fixé à la platine 100. Sur le dessus de la plaquette 70, à faible distance de celle-ci et à un niveau légèrement supérieur à la hauteur de la bandelette (b) transportée par la pince 2, est disposée une languette de retenue 72. Juste devant la plaquette 70, il est prévu dans la platine 100 une ouverture 101 par exemple circulaire. La pince 2 étant déplacée au poste d'éjection 7 (flèche $I_1$, figure 11), le bord avant de la bandelette vient buter contre la plaquette inclinée 70, ce qui oblige la bandelette à se cintrer vers le haut et à passer sous la languette 72. Dans le même temps, la face inférieure de la mâchoire 25 vient en vis à vis de l'ouverture 101, ce qui provoque son ouverture par basculement (flèche $I_2$). La bandelette (b) n'est donc plus retenue positivement par la pince, mais se trouve simplement en appui contre le fond de l'interstice séparant les deux mâchoires 23, 25. Si on provoque maintenant le recul de la pince 2 (flèche $I_3$, figure 12), la bandelette (b) qui se trouve arc-boutée sous la languette 72 se déploie par sa propre élasticité, et quitte la pince 2 ; elle tombe alors par gravité à travers l'ouverture 101 (bandelette (b') représentée en traits interrompus) à l'intérieur d'un bac de récupération, ou autre récipient 73, avantageusement prévu sous cette ouverture 101.

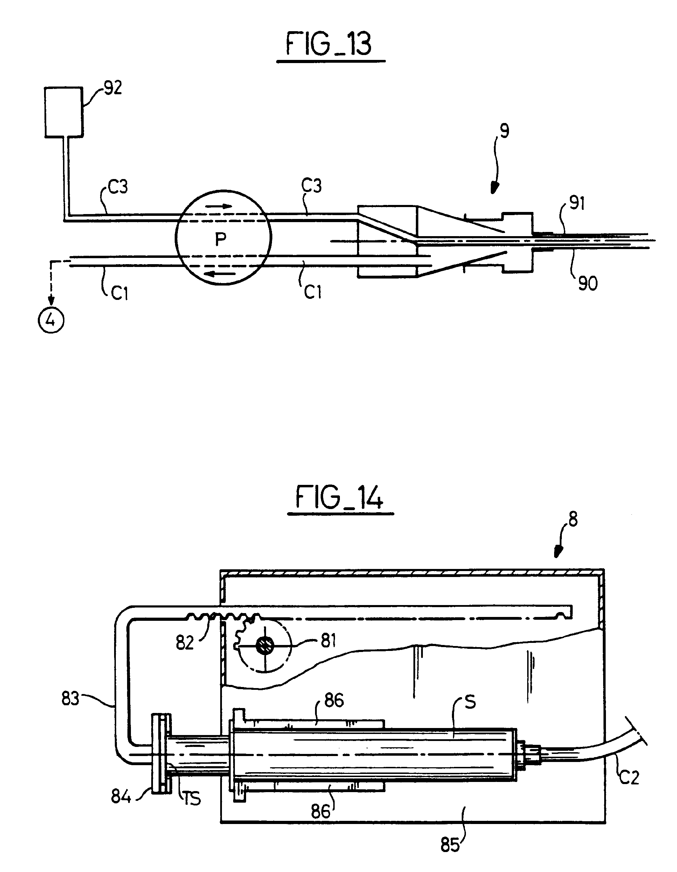

La figure 13 représente un cathéter dit "double lumière" particulièrement adapté pour le prélèvement de l'échantillon de sang qui doit être amené au poste 4. Il permet en effet de prélever par le cathéter ($C_1$) non pas du sang pur mais du sang hépariné, c'est-à-dire un mélange de sang et d'environ 10 % d'héparine. Le cathéter ($C_1$) débouche dans un corps creux ou bouchon 9, qui est traversé complètement par un cathéter ($C_3$), de plus petit diamètre. Sur le bouchon 9 est raccordé un cathéter 90 destiné à être enfoncé dans la veine du patient. Ce cathéter 90 entoure l'extrémité 91 du cathéter ($C_3$) et a une longueur légèrement supérieure à celle de cette extrémité. Les cathéters ($C_3$, $C_1$) passent dans une pompe péristaltique unique (P) de type connu, à deux cassettes tel que par exemple une pompe comercialisée sous le nom commercial ISMATEC. Cette pompe assure le cheminement lent et régulier de l'héparine dans le cathéter ($C_3$) depuis un récipient 92 jusqu'à l'extrémité du cathéter 91, et simultanément l'acheminement du sang hépariné du cathéter 90 via le bouchon 9 et le cathéter ($C_1$) vers le poste 4. Il est naturellement important que l'héparine ne soit pas injectée dans le corps du patient, et c'est pourquoi le cathéter 91 est plus court que le cathéter 90. Le mélange se fait immédiatement après le prélèvement ; par rapport au sang pur, un tel mélange présente l'avantage de ne pas se coaguler, ce qui empêche le bouchage des cathéters.

L'unité d'injection de l'insuline représentée à la figure 14 comprend un boîtier support 8, également monté sur la platine 100. Sur le dessus de ce boîtier est prévu un berceau semi-cylindrique 86 apte à recevoir par encastrement une seringue (S). Celle-ci est remplie d'insuline au début de l'opération. Le dispositif comprend un coulisseau 83 équipé d'un adaptateur 84 apte à être emcliqueté sur la tête de piston (TS) de la seringue. Le coulisseau 83 est solidaire d'une crémaillère 82 logée à l'intérieur du boîtier et guidée en translation dans celui-ci, parallèlement à l'axe de la seringue. Un pignon 81 qui est entraîné par un moto-réducteur approprié (non représenté, également logé dans le boîtier) permet de déplacer la crémaillère 82, sur une course bien définie, et donc de déplacer de manière correspondante le piston de la seringue, ce qui permet d'envoyer une certaine dose d'insuline au patient via le cathéter ($C_2$).

Nous allons maintenant expliquer de quelle manière le pancréas artificiel qui vient être décrit est utilisé.

Les données (d) relatives au patient (M) sont tout d'abord introduites par le thérapeute dans l'unité (U). Ces données sont notamment le nom, le poids, l'âge du patient, une estimation de son besoin insulinique journalier en sous-cutané, et l'objectif glycémique que l'on souhaite atteindre. Est programmée également-

ment la fréquence à laquelle les prélèvements vont être effectués, par exemple au cours d'une journée, ainsi que la répartition dans le temps des prélèvements, la fréquence pouvant être plus élevée au moment des repas. A titre indicatif les prélèvements pourront se faire tous les 7,5 minutes ou toutes les 15 minutes. L'unité (U) va donc piloter automatiquement et séquentiellement l'ensemble du processus selon la fréquence et le mode de répartition sélectionnés.

Un cycle de prélèvement de sang, de mesure de la glycémie, et d'injection d'insuline, se passe de la manière suivante :

Des bandelettes vierges se trouvant stockées au poste 3, l'unité (U) commande la mise en route des moto-réducteurs 16 et 22 de manière à d'une part positionner convenablement la tourelle 10 pour que l'axe (X-X′) de la pince 2 se trouve en regard du poste 3, et d'autre part à déplacer la pince pour qu'elle arrive à proximité de ce poste (flèche i, figure 5a). Simultanément, la molette 33 est mise en rotation de manière à délivrer une bandelette (b) qui est alors saisie par la pince 2 comme cela a déjà été expliqué plus haut. Le transporteur est alors mis à nouveau en mouvement, de telle sorte que la bandelette (b), toujours portée par la pince 2, soit amenée au poste 4. Un peu avant l'arrivée de la bandelette (b) à ce poste, la pompe péristlatique (P) a été mise en marche, de manière à prélever un peu de sang hépariné dans le bras du patient. La dose prélevée est envoyée par la pompe dans le conduit 42. Pour être certain que le sang qui arrive dans le conduit 42 vient d'être prélevé, on laisse passer librement une certaine quantité de sang qui ne sera pas utilisé pour le test. Cette quantité de sang, par exemple de 15 gouttes environ se trouvait en effet préalablement dans le cathéter (C$_1$) et correspond à un prélèvement fait antérieurement. Ce sang non utilisé tombe dans le récipient 44 en traversant l'ouverture 430 de l'élément d'appui 43, il est prévu un compteur de gouttes non représenté, de type usuel, de sorte que l'unité (U) peut piloter le dispositif pour que ce soit une goutte de sang prédéterminée (par exemple la seizième) qui tombe sur la zone réactive (z) de la bandelette (b) mise en place au poste 4. Il est prévu un détecteur de bandelettes de type connu par exemple optoélectronique, qui permet à l'unité (U) de vérifier qu'une bandelette (b) est bien présente à ce poste ; de même il est prévu un détecteur de gouttes lui permettant de s'assurer qu'une goutte tombe bien sur la zone réactive de la bandelette. En cas de défaut, un signal d'erreur s'affiche sur l'écran (E) et une alarme est déclenchée.

Lorsque la goutte de sang a été placée sur la bandelette (b), celle-ci est transférée par la pince 2 au poste d'essuyage 5. Comme on l'a déjà dit plus haut, l'arrivée de la pince à ce poste provoque le soulèvement de la bande d'essuyage 57, si bien que la zone (z) peut se positionner entre les sabots presseurs 55 et 52 ; la bobine 50′ est alors mise en rotation (flèche j$_1$), ce qui provoque un déplacement de la bande de papier 57 (flèche j$_2$), et l'essuyage de la goutte de sang.

On notera que l'entraînement de la bobine réceptrice 50′ engendre un couple de basculement de la plaque 53 (flèche j$_3$) qui tend à améliorer l'action de pressage de la bande de papier 57 contre la bandelette. Ce résultat est obtenu grâce au fait que la plaque 53 est articulée sur le même axe 500′ que la bobine motrice 50′. Ceci évite d'utiliser des ressorts de rappel.

A ce moment, l'électro-aimant 60 est déclenché et agit sur l'interrupteur du boîtier 62 pour mettre sous tension le dispositif de lecture. La lecture de la couleur de la zone réactive doit se faire au bout d'un délai prédéterminé après l'essuyage, par exemple d'une minute. Le transporteur est donc actionné de manière à amener la bandelette au poste 9 dans ce délai, la bandelette étant introduite par la pince mobile 2 dans la fente 64 du dispositif de lecture. Le lecteur glycémique possède en réalité deux systèmes optiques de lecture distincts qui évaluent indépendamment les deux zones réactives (z$_1$) et (z$_2$). Les résultats de ces mesures sont transmis par la ligne (L$_1$) à l'unité (U). Une fois la lecture terminée, la bandelette est extraite du boîtier 62, toujours par la pince 2, et la tourelle est mise en rotation en sens contraire, pour revenir au poste de distribution 3 de nouvelles bandelettes. Cependant en cours de route, la tourelle s'arrête en vis à vis du poste d'éjection 7. A ce poste, qui, dans le mode de réalisation représenté est situé entre les postes 4 et 5, la bandelette usagée est évacuée, comme cela a déjà été expliqué en détail plus haut en référence aux figures 10, 11 et 12.

Le transporteur 2, revient alors à vide au point de départ c'est-à-dire au poste 3, dans l'attente de l'ordre émanant de l'unité (U) pour un nouveau cycle.

Les résultats de la lecture faite au poste 6 sont calculés et analysés par l'unité (U) ; ces résultats sont affichés sur l'écran (E) et imprimés sur papier par l'imprimante (I) de telle manière que le thérapeute est à tout moment renseigné sur l'évolution de la situation. L'unité (U) calcule par ailleurs la dose d'insuline qui, en fonction de la mesure de glycémie qui vient être faite, doit être administrée au patient. La dose est effectivement administrée par actionnement du moto-réducteur 80 qui, par l'intermédiaire du pignon 81 et de la crémaillère 82 va déplacer le piston de la seringue (S) sur une course bien déterminée.

Le pancréas artificiel qui vient d'être décrit est très simple à utiliser, et requiert un personnel réduit. Il est très fiable du fait qu'il utilise des composants simples et connus ; les disfonctionnements éventuels sont aisément identifiables et peuvent être corrigés rapidement. Dans un mode de réalisation avantageux, toutes les unités constitutives de ce pancréas artificiel peuvent être prévues sur un support unique, ce support ayant l'allure d'un piètement supportant une console informatique comprenant un clavier et un écran (moniteur). Le clavier, situé devant l'écran, peut avantageusement consister en un couvercle articulé sur un boîtier dans lequel est logée la platine 100 et l'ensemble des postes de mesure, l'unité d'injection (pousse-seringue) et la pompe péristaltique. Ainsi, en position d'utilisation (couvercle/clavier rabattu sur le boîtier), l'ensemble des composants mécaniques est caché et protégé, tout en étant aisément accessible.

Diverses variantes du dispositif sont bien entendu possible.

Ainsi, dans l'une de ces variantes, il serait possible de supprimer le sabot presseur supérieur 55 du poste d'essuyage, les galets de renvoi 56, 56' étant dans ce cas adaptés pour mettre convenablement la bande de papier en appui contre le sabot inférieur 52.

Dans le cas où le pancréas artificiel est prévu pour injecter du glucose, le dispositif d'injection à seringue et pousse-seringue est de préférence remplacé par une pompe péristaltique reliée à un récipient de glucose. Une telle pompe permet en effet d'administrer au patient des doses relativement importantes de produit et donc de travailler sous une dilution élevée (ce qui évite la brûlure des veines par le glucose).

Du reste, la pancréas artificiel selon l'invention pourrait être équipé à la fois d'un système d'injection d'insuline et d'un système d'injection de glucose.

## Revendications

1. Pancréas artificiel, du type comprenant une unité de prélèvement automatique et périodique d'échantillons de sang dans le corps d'un patient (M), une unité de mesure du taux de glucose présent dans cet échantillon (glycémie), une unité (8) d'injection d'une dose d'insuline ou de glucose dans le corps du patient, et une unité (U) de calcul et de traitement informatique apte à déterminer la quantité d'insuline ou de glucose devant être administrée au patient en fonction du taux de glucose mesuré par l'unité de mesure, et à commander de manière correspondante l'unité d'injection (8), caractérisé en ce que la mesure de la glycémie est effectuée par lecture de la couleur de la zone (z) d'une bandelette (b) réactive à la glycémie, l'unité de mesure comprenant à cet effet :

a) un poste (3) de distribution de bandelettes (b) ;

b) un poste (4) dans lequel une goutte de sang fournie par ladite unité de prélèvement est disposée sur la zone réactive (z) d'une bandelette (b) ;

c) un poste (5) d'essuyage de la zone (z) ;

d) un poste (6) de lecture optique de la couleur prise par la zone (z) ;

e) un poste (7) d'évacuation de la bandelette ;

f) un dispositif de transport (1, 2) apte à acheminer individuellement les bandelettes (b) de poste en poste.

2. Pancréas artificiel selon la revendication 1, caractérisé en ce que le dispositif de transport comprend une pince (2) dont les mâchoires (23, 25) sont adaptées pour saisir et retenir une bandelette (b), ladite pince (2) étant montée mobile en translation selon un axe sensiblement horizontal (X-X') dans une tourelle rotative (10) d'axe vertical (Z-Z').

3. Pancréas artificiel selon l'une des revendications 1 ou 2, caractérisé en ce que le poste (3) de distribution des bandelettes comprend un réceptacle (30) dans lequel les bandelettes (b) sont empilées horizontalement sous une certaine pression, une molette d'entraînement rotative (33) prévue au niveau du fond (31) de ce réceptacle étant adaptée pour faire sortir du réceptacle (30), par une ouverture (32), la bandelette inférieure de la pile.

4. Pancréas artificiel selon l'une des revendications 2 ou 3, caractérisé en ce que l'une (23) des mâchoires de ladite pince (2) est munie d'une languette élastique (24) apte à bloquer la bandelette (b).

5. Pancréas artificiel selon l'une des revendications 1 à 4, caractérisé en ce que le poste (4) dans lequel une goutte de sang est déposée sur la zone réactive de la bandelette (b) comprend une plaque-support (40) traversée par un petit conduit (42) d'amenée du sang, et un élément d'appui (43) de la bandelette, disposé sous ce petit conduit (42).

6. Pancréas artificiel selon l'une des revendications 1 à 5, caractérisé en ce que le poste (5) d'essuyage comprend un bâti (51) équipé d'une paire de bobines (50, 50') - respectivement distributrice et réceptrice - d'une bande de papier absorbant (57), des moyens étant prévus pour entraîner en rotation l'une au moins (50') des bobines sur une certaine course.

7. Pancréas artificiel selon la revendication 6, caractérisé en ce que ladite bande (57) est guidée par des galets (56, 56') pour passer entre un sabot presseur inférieur (52) et un sabot presseur supérieur (55), entre lesquels vient se positionner la zone réactive de la bandelette traitée.

8. Pancréas artificiel selon la revendication 7, caractérisé en ce que ladite bande (57) et le sabot presseur supérieur (55) sont portés par une plaque (53) qui est articulée sur le bâti (51) autour d'un axe horizontal (500'), cette plaque (53) étant solidaire d'une came (54) apte à coopérer avec un organe de commande (27) équipant la pince (2) pour provoquer le soulèvement temporaire du sabot supérieur (55) et de la bande (57) lors de la mise en place de la zone réactive de la bandelette entre les deux sabots (52-55) et lors du retrait de cette bandelette

9. Pancréas artificiel selon l'une des revendications 1 à 7, caractérisé en ce que le poste (6) de lecture optique de la couleur prise par la zone réactive (z) de la bandelette (b) comprend un boîtier de lecture (62) pourvu d'une fente (64) tournée vers le dispositif transporteur (1, 2) et orientée de manière à se trouver sur la trajectoire de la bandelette (b).

10. Pancréas artificiel selon l'une des revendications 2 à 9, caractérisé en ce que la pince (2) comprend une mâchoire supérieure (23) fixe et une mâchoire inférieure (25) articulée autour d'un axe horizontal (26), la fermeture de la pince étant normalement assurée par l'appui de cette mâchoire inférieure (25) sur la platine de montage (100) de l'ensemble des postes.

11. Pancréas artificiel selon la revendication 10, caractérisé en ce que le poste (7) d'évacuation de la bandelette comprend une plaquette inclinée (70) munie d'une languette (72) de retenue de la bandelette (b), cette plaquette (70) étant disposée à proximité d'une ouverture (101) ménagée dans la platine de montage (100), ladite ouverture réalisant d'une part l'ouverture de la pince (2) par basculement de la mâchoire inférieure (25) et permettant d'autre part le passage des bandelettes évacuées (b').

**Claims**

1. Artificial pancreas of the type comprising a unit for automatic and periodical withdrawal of blood samples from the body of a patient (M), a unit for measuring the level of glucose present in this sample (glycemia), a unit (8) for injection of a dose of insulin or glucose into the body of the patient, and a unit (U) for calculation and data processing able to determine the amount of insulin or glucose which is to be administered to the patient as a function of the glucose level measured by the measurement unit, and to control the injection unit (8) in a corresponding manner, characterized in that the measurement of the glycemia is carried out by reading of the color of the zone (z) of a strip (b) reactive to glycemia, the measurement unit comprising to this effect:

a) a station (3) for distribution of strips (b);
b) a station (4) in which a blood droplet supplied by the said sampling unit is arranged on the reactive zone (z) of a strip (b);
c) a station (5) for wiping the zone (z);
d) a station (6) for optical reading of the color taken by the zone (z);
e) a station (7) for removing the strip;
f) a transportation device (1, 2) for conveying the strips (b) individually from station to station.

2. Artificial pancreas according to claim 1, characterized in that the transportation device comprises a pincer (2) whose jaws (23, 25) are designed to grip and retain a strip (b), the said pincer (2) being mounted movable in translation along a substantially horizontal axis (X-X') in a rotatory turret (10) of vertical axis (Z-Z').

3. Artificial pancreas according to one of claims 1 or 2, characterized in that the strip distribution station (3) comprises a container (30) in which the strips (b) are stacked horizontally under a certain pressure, a rotatory drive knurled wheel (33) provided at the level of the base (31) of this container being designed to remove from the container (30), through an opening (32), the bottom strip of the stack.

4. Artificial pancreas according to one of claims 2 or 3, characterized in that one (23) of the jaws of the said pincer (2) is provided with a small elastic tongue (24) for blocking the strip (b).

5. Artificial pancreas according to one of claims 1 to 4, characterized in that the station (4) in which a blood droplet is deposited on the reactive zone of the strip (b) comprises a support plate (40) passed through by a small conduit (42) for delivery of the blood, and an element (43), for bearing of the strip, arranged under this small conduit (42).

6. Artificial pancreas according to one of claims 1 to 5, characterized in that the wiping station (5) comprises a frame (51) equipped with a pair of reels (50, 50') for distributing and receiving, respectively, a band of absorbent paper (57), means being provided for driving at least one (50') of the reels in rotation over a certain course.

7. Artificial pancreas according to claim 6, characterized in that the said band (57) is guided by rollers (56, 56') so as to pass between a lower pressing block (52), and an upper pressing block (55), between which the reactive zone of the treated strip takes up a position.

8. Artificial pancreas according to claim 7, characterized in that the said band (57) and the upper pressing block (55) are borne by a plate (53) which is articulated on the frame (51) about a horizontal axis (500'), this plate (53) being integral with a cam (54) for cooperating with a control member (27) equipped on the pincer (2) for bringing about the temporary lifting of the upper block (55) and the band (57) during the positioning of the reactive zone of the strip between the two blocks (52-55) and during withdrawal of this strip.

9. Artificial pancreas according to one of claims 1 to 7, characterized in that the station (6) for optical reading of the color taken by the reactive zone (z) of the strip (b) comprises a reading casing (62) provided with a slot (64) turned towards the transporter device (1, 2) and oriented in such a way as to be situated in the trajectory of the strip (b).

10. Artificial pancreas according to one of claims 2 to 9, characterized in that the pincer (2) comprises a fixed upper jaw (23) and a lower jaw (25) articulated about a horizontal axis (26), the closure of the pincer being normally ensured by this lower jaw (25) bearing on the assembly deck (100) for the set of stations.

11. Artificial pancreas according to claim 10, characterized in that the station (7) for removal of the strip comprises an inclined plate (70) provided with a small tongue (72) for retaining the strip (b), this plate (70) being arranged in the proximity of an opening (101) made in the assembly deck (100), the said opening on the one hand causing the pincer (2) to open by means of the swinging of the lower jaw (25) and on the other hand permitting the passage of the removed strips (b').

**Patentansprüche**

1. Künstliche Bauchspeicheldrüse des Typs, der eine Einheit zur automatischen und periodischen Entnahme von Blutproben aus dem Körper des Patienten (M) enthält, eine Meßeinheit für den in dieser Probe enthaltenen Glukoseanteil (Blutzucker), eine Einheit (8) für die Injektion einer Dosis Insulin oder Glukose in den Körper des Patienten, und eine Datenrechen- und verarbeitungseinheit (U), die geeignet ist, das Quantum Insulin oder Glukose zu bestimmen, das dem Patienten verabreicht werden muß in Abhängigkeit des von der Meßeinheit gemessenen Glukosespiegels, und die Injektionseinheit (8) in entsprechender Weise zu steuern,
dadurch **gekennzeichnet,**
daß die Blutzuckermessung durch das Feststellen der Farbe der Zone (z) eines auf Blutzucker reagierenden Streifens (b) erfolgt, wobei die Meßeinheit zu diesem Zweck enthält:
a) eine Verteilstation (3) für die Streifen (b);
b) eine Station (4), in der ein von der genannten Entnahmeeinheit gelieferter Blutstropfen auf die reaktive Zone (z) eines Streifens aufgetropft wird;
c) eine Trockenstation (5) für die Zone (z) ;
d) eine optische Lesestation (6) für das Erkennen der Farbe, welche die Zone (z) angenommen hat;
e) eine Station (7) für die Beseitigung des Streifens;
f) eine Transportvorrichtung, geeignet die Streifen einzeln von Station zu Station zu transportieren.

2. Künstliche Bauchspeicheldrüse nach Anspruch 1, dadurch gekennzeichnet, daß die Transportvorrichtung eine Zange (2) enthält, deren Klemmbacken (23, 25) geeignet sind, einen Streifen (b) zu ergreifen und festzuhatten, wobei genannte Zange (2) beweglich montiert ist für Translationsbewegungen in einer im wesentlichen horizontalen Achse (X-X') in einem drehbaren Halter (10) mit vertikaler Achse (Z-Z').

3. Künstliche Bauchspeicheldrüse nach einem der Ansprüche 1 oder 2, dadurch gekennzeichnet, daß die Verteilstation (3) für Streifen einen Aufnahmebehälter (30) enthält, in der die Streifen (b) horizontal unter einem gewissen Druck aufgestapelt sind, wobei auf der Höhe des Bodens (31) dieses Aufnahmebehälters eine drehbare Antriebsrolle (33) vorgesehen ist, um durch eine Öffnung (32) den untersten Streifen des Stapels aus dem Aufnahmebehälter (30) austreten zu lassen.

4. Künstliche Bauchspeicheldrüse nach einem der Ansprüche 2 oder 3, dadurch gekennzeichnet, daß einer (23) der Klemmbacken der genannten Zange (2) eine elastische Zunge (24) enthält, geeignet den Streifen (b) festzuhalten.

5. Künstliche Bauchspeicheldrüse nach einem der Ansprüche 1 bis 4, dadurch gekennzeichnet, daß die Station (4), in welcher der Blutstropfen auf die reaktive Zone des Streifens (b) aufgetropft wird, einen Träger (40) enthält, den ein Zuführungsröhrchen (42) für das Blut durchquert, und ein Auflageelement (43) für den Streifen, angeordnet unter diesem Zuführungsröhrchen (42).

6. Künstliche Bauchspeicheldrüse nach einem der Ansprüche 1 bis 5, dadurch gekennzeichnet, daß die Trockenstation (5) einen Aufbau (51) enthält, der ein Spulenpaar (50, 50') aufweist - Abroller bzw. Aufroller - eines absorbierenden Papierbandes (57), wobei Mittel vorgesehen sind, zumindest eine (50') der Spulen für eine gewisse Strecke anzutreiben.

7. Künstliche Bauchspeicheldrüse nach Anspruch 6, dadurch gekennzeichnet, daß das genannte Band (57) mittels Umlenkrollen (56, 56') durchgeführt wird zwischen einem unteren Preßschuh (52) und einem oberen Preßschuh (55), zwischen denen die reaktive Zone des zu behandelnden Streifens zu liegen kommt.

8. Künstliche Bauchspeicheldrüse nach Anspruch 7, dadurch gekennzeichnet, daß das genannte Band (57) und der obere Preßfuß (55) getragen werden von einer Platte (53), die gelenkig gelagert ist auf dem Gestell (51) um eine horizontale Achse (500'), wobei an dieser Platte (53) eine Nocke (54) befestigt ist, die zusammenwirkt mit einem auf der Zange (2) befestigten Steuerungsorgan (27), um das vorübergehenden Anheben des oberen Preßschuhs (55) und des Bands (57) zu bewirken, während des Einführens des Streifens zwischen die beiden Preßschuhe (52, 55) und während des Zurückziehens dieses Streifens.

9. Künstliche Bauchspeicheldrüse nach einem der Ansprüche 1 bis 7, dadurch gekennzeichnet, daß die optische Lesestation (6) der von der reaktiven Zone (z) des Streifens (b) angenommenen Farbe ein mit einem Schlitz (64) versehenes Lesegehäuse (62) enthält, der Transportvorrichtung (1, 2) zugekehrt und so ausgerichtet, daß es sich auf der Bahn des Streifens (b) befindet.

10. Künstliche Bauchspeicheldrüse nach einem der Ansprüche 2 bis 9, dadurch gekennzeichnet, daß die Zange (2) einen oberen feststehenden Klemmbacken (23) enthält und einen unteren (25), schwenkbar gelagert um eine horizontale Achse (26), wobei die Schließung der Zange normalerweise erfolgt durch das Aufliegen dieser unteren Klemmbacke (25) auf der gemeinsamen Montageplatte (100) aller Stationen.

11. Künstliche Bauchspeicheldrüse nach Anspruch 10, dadurch gekennzeichnet, daß die Streifenbeseitigungsstation (7) eine geneigte Platte 70 mit einer Rückhaltezunge (72) für den Streifen (b) enthält, wobei diese Platte (70) in der Nähe einer in der Montageplatte (100) angebrachten Öffnung (101) angeordnet ist, wobei die genannte Öffnung einerseits das Öffnen der Zange (2) durch Herunterklappen der unteren Klemmbacke (25) möglich macht und andererseits die Passage der beseitigten streifen (b') zuläßt.

# FIG_1

EP 0 381 736 B1

FIG_2

FIG_3

FIG_4

FIG_5

FIG_5a

## FIG_6

## FIG_7

## FIG_8

## FIG_9

## FIG_10

## FIG_11

## FIG_12

## FIG_13

## FIG_14